(19) Europäisches Patentamt
European Patent Office
Office européen des brevets

(11) **EP 1 392 193 B1**

## (12) EUROPÄISCHE PATENTSCHRIFT

(45) Veröffentlichungstag und Bekanntmachung des Hinweises auf die Patenterteilung:
**09.12.2009 Patentblatt 2009/50**

(21) Anmeldenummer: **02748733.9**

(22) Anmeldetag: **04.06.2002**

(51) Int Cl.:
***A61C 1/00*** *(2006.01)*

(86) Internationale Anmeldenummer:
**PCT/EP2002/006136**

(87) Internationale Veröffentlichungsnummer:
**WO 2002/098315 (12.12.2002 Gazette 2002/50)**

(54) **MEDIZINISCHES ODER DENTALMEDIZINISCHES INSTRUMENT UND/ODER VERSORGUNGSGERÄT UND/ODER PFLEGEGERÄT FÜR DAS MEDIZINISCHE ODER DENTALMEDIZINISCHE INSTRUMENT**

MEDICAL OR DENTAL INSTRUMENT AND/OR SUPPLY UNIT AND/OR CARE UNIT FOR THE MEDICAL OR DENTAL INSTRUMENT

INSTRUMENT MEDICAL OU DE MEDECINE DENTAIRE ET/OU APPAREIL D'ALIMENTATION ET/OU APPAREIL DE SOIN POUR LEDIT INSTRUMENT MEDICAL OU DE MEDECINE DENTAIRE

(84) Benannte Vertragsstaaten:
**AT CH DE FI IT LI**

(30) Priorität: **07.06.2001 DE 10127772**

(43) Veröffentlichungstag der Anmeldung:
**03.03.2004 Patentblatt 2004/10**

(60) Teilanmeldung:
**09151926.4 / 2 067 451**

(73) Patentinhaber: **Kaltenbach & Voigt GmbH**
**88400 Biberach / Riss (DE)**

(72) Erfinder:
• **BEIER, Stefan**
**88400 Biberach (DE)**
• **EIBOFNER, Eugen**
**88400 Biberach-Mettenberg (DE)**

• **HECKENBERGER, Hans**
**88433 Assmannshardt (DE)**
• **STROHMAIER, Ernst**
**88427 Bad Schussenried (DE)**
• **IMHOF, Armin**
**88400 Biberach (DE)**

(74) Vertreter: **Schmidt-Evers, Jürgen**
**Mitscherlich & Partner**
**Patent- und Rechtsanwälte**
**Sonnenstrasse 33**
**80331 München (DE)**

(56) Entgegenhaltungen:
**WO-A-98/06338      DE-A- 3 726 262**
**DE-A- 19 729 177    US-A- 5 807 521**
**US-A- 6 092 722     US-A- 6 117 285**

## Beschreibung

Die Erfindung betrifft ein medizinisches oder dentalmedizinisches Instrument oder ein Versorgungsgerät und/oder eine Pflegevorrichtung für medizinische oder dentalmedizinische Instrumente nach dem Oberbegriff der Ansprüche 1, 4 oder 6.

Bei einem medizinischen oder dentalmedizinischen Instrument handelt es sich um einen Gegenstand, der zur Bearbeitung des menschlichen oder tierischen Körpers oder Teilen (Prothesen) davon eingesetzt wird und dabei in Kontakt mit dem Körper kommt und dabei verunreinigt und/oder mit Krankheitserregern kontaminiert werden kann. Dies insbesondere dann, wenn das Instrument mit einem Sekret oder einer Flüssigkeit des Körpers in Kontakt kommt. Es ist deshalb erforderlich, nach der Bearbeitung eines Patienten bzw. vor der Bearbeitung eines nächsten Patienten das Instrument zu reinigen und/oder zu desinfizieren und/oder sterilisieren. Die jeweilige Maßnahme ist davon abhängig, zum einen wie intensiv der Kontakt des Instruments mit dem Körper war und zum anderen welche Bearbeitungsmaßnahme durchgeführt worden ist.

Die Ausgestaltung und Funktion eines vorliegenden Instruments kann außerdem sehr unterschiedlich sein. Insbesondere bei einem vorgenannten Instrument, dessen Werkzeug durch einen Antrieb bewegt wird, bedarf es in gewissen Zeitabständen einer mechanischen Wartung des wenigstens einen Instruments, um dessen Funktionsfähigkeit aufrecht zu erhalten und Verschleiß und Funktionsstörungen zu vermeiden. Es gibt Instrumente, bei denen unabhängig davon, ob sie im Funktionsbetrieb verunreinigt und/oder kontaminiert werden, die Vorschrift besteht, das Instrument in gewissen Zeitabständen, z. B. nach bestimmten Betriebsstunden, zu warten. Die vorbeschriebenen Maßnahmen zur Aufrechterhaltung der Einsatzbereitschaft des wenigstens einen Instruments werden im Folgenden mit dem Begriff "Pflege" bezeichnet. Es gibt deshalb für ein vorliegendes Instrument eine Benutzungsphase, in der es auf den Körper wenigstens zeitweise einwirkt, und eine Pflegephase, in der auf das Instrument zur Aufrechterhaltung seiner Einsatzbereitschaft eingewirkt wird.

In der Benutzungsphase ist das Instrument mit einem medizinischen oder dentalmedizinischen Versorgungsgerät verbunden. Gemäß dem derzeitigen Standard weist das Versorgungsgerät eine elektronische Steuereinrichtung auf, von der sich eine biegsame bzw. flexible Versorgungsleitung erstreckt, mit der das Instrument durch eine lösbare Kupplung verbunden ist. Das Instrument bzw. sein Werkzeug wird für seine Funktion durch die Versorgungsleitung hindurch vom Versorgungsgerät versorgt bzw. gesteuert. Hierbei kann es sich um die Zuführung von Antriebsenergie und/oder Bearbeitungsmitteln handeln.

Dem Versorgungsgerät kann nur ein oder können mehrere unterschiedliche Instrumente zugeordnet sein, die wahlweise durch die Kupplung mit der Versorgungsleitung verbindbar sind. Hierbei können je nach Art des Instruments unterschiedliche Antriebsmittel vorhanden sein bzw. zugeführt werden. Ein Versorgungsgerät dieser Art ist z. B. in der DE 4 410 276 A1 beschrieben.

In der DE 42 20 522 A1 ist ein System zur Kenntlichmachung verschiedener Verbraucher, beispielsweise zahnärztliche Instrumente, gegenüber einer zu einem zahnärztlichen Behandlungsplatz gehörenden Energie-Versorgungseinheit beschrieben, mit an einer Versorgungsleitung vorgesehenen Codierungsmitteln zur Identifizierung eines der Versorgungsleitung zugeordneten Instruments und mit einer zu der Energie-Versorgungseinheit gehörenden Auswerteschaltung, die durch elektrische Verbindungsleitungen mit den Codierungsmitteln verbunden ist. Die elektrischen Verbindungsleitungen erstrecken sich durch die Versorgungsleitung.

Aus der DE 19 729 177 A1 ist ein ärztliches Versorgungsgerät zu entnehmen, bei dem mit Hilfe einer elektronischen Steuereinrichtung ein an einem Antriebsmotor ankoppelbares Instrument mit Werkzeug versorgt und gesteuert werden kann. In dem Steuergerät sind herstellerseitig fest vorgegebene Betriebsprogramme für den Betrieb bestimmter Instrumente und Werkzeuge gespeichert. Zudem können benutzerspezifische Betriebsprogramme erstellt werden. Des weiteren kann mit Hilfe einer Auswahltaste ein Einstellmodus abgerufen werden, in dem der Benutzer beliebig in den Betriebsablauf eingreifen und die Betriebsparameter beliebig verändern kann.

In der DE 199 13 962 A1 ist eine Pflegevorrichtung für zahnärztliche Handstücke beschrieben, mit einem Gehäuse, das einen Pflegeraum umschließt. Im Pflegeraum ist wenigstens ein Kupplungsteil zur Aufnahme des einen Endes eines Instruments angeordnet, wobei sich eine Pflege-Zuführungsleitung zum Kupplungsteil erstreckt. Zwecks Gewährleistung einer einfachen Ausgestaltung und einer sicheren Funktion ist dem Kupplungsteil ein Sensor zugeordnet, der durch das Instrument in dessen Pflegestellung zwecks Abgabe eines Signals an eine Steuereinrichtung des Pflegegeräts aktiviert wird, worauf die Steuereinrichtung das Zuführungssystem für Pflegemittel ansteuert.

Dokument WO98/6338 offenbart ein medizinisches Instrument mit Speicherelementen, die mit einem Versorgungsgerät und einem Pflegegerät ausgelesen werden können.

Eine Codierung an einem vorliegenden Instrument kann Identifizierungs- und/oder Betriebsparameterdaten enthalten und stellt somit ein Speicherelement für solche Daten dar, die bei einer Kopplung des Instruments mit einem medizinischen oder dentalmedizinischen Versorgungsgerät vom Gerät abrufbar sind und zur Steuerung der Versorgung des Instruments benutzt werden können.

Der Erfindung liegt die Aufgabe zugrunde, bei einem Instrument nach dem Oberbegriff des Anspruches 1 die Einsatzmöglichkeiten zu erweitern.

Diese Aufgabe wird durch die Merkmale des Anspruches 1 gelöst.

Bei erfindungsgemäßen Instrument ist das Speicherelement zum Überschreiben der Daten durch andere Daten ausgebildet. Hierdurch ist es möglich, den Datenspeicher am Instrument zu verändern. Folglich kann das Instrument als Datenleiter für die geänderten Daten benutzt werden, woraus sich wesentliche Vorteile ergeben. Zum einen ist das Instrument zumindest für den Zeitabschnitt, in dem es vom Gerät getrennt ist, vom Datenspeicher des Gerätes unabhängig. Außerdem kann das Instrument bei einer wahlweisen Kupplung mit mehreren Geräten die geänderten Daten auf das jeweilige Gerät übertragen, so daß es einer Datenübertragung direkt zwischen den Geräten nicht bedarf. Darüber hinaus führt die Erfindung auch zu dem Vorteil, daß das Instrument aufgrund der Änderbarkeit seiner Daten nicht nur an unterschiedliche Daten anpaßbar ist, sondern sich auch dazu eignet, Daten zu sammeln, z.B. bei einander folgenden Betriebsphasen. Hierbei kann in das Speicherelement mindestens ein Parameter aus einer vorherigen Betriebsphase eingeschrieben und zur Steuerung der nächsten Betriebsphase ausgelesen werden. Es können Abhängigkeiten zwischen Betriebsphasen geschaffen werden und eine weitere Betriebsphase nur dann durchgeführt werden, wenn eine vorherige Betriebsphase durchgeführt worden ist. Eine diesbezügliche Datenabfrage kann beim Kuppeln des Instruments mit einem Gerät zur Durchführung der weiteren Betriebsphase abgerufen werden. Dies ist insbesondere dann wichtig, wenn Betriebsphasen nur dann durchgeführt werden sollen bzw. dürfen, wenn die vorherige Betriebsphase durchgeführt worden ist. Dieses Erfordernis ist bei der Pflege und/oder Wartung des Instruments gegeben. Außerdem eignet sich das Instrument zu einer weiterführenden Registrierung seiner Daten, so daß der Betriebsablauf des Instruments nachvollzogen werden kann.

Der Erfindung liegt im weiteren die Aufgabe zugrunde, bei einem Versorgungsgerät nach dem Oberbegriff des Anspruches 4 die Datenleitung zu verbessern bzw. zu erweitern.

Diese Aufgabe wird durch die Merkmale des Anspruches 4 gelöst. Beim erfindungsgemäßen Versorgungsgerät nach Anspruch 4 ist vorzugsweise der Datenempfänger oder eine Lesevorrichtung auch ein Datensender zum Übertragen von Daten auf das Speicherelement. Hierdurch lassen sich Daten durch die Versorgungsleitung abgeben und es läßt sich der Datenspeicher des Versorgungsgeräts zumindest für die Zeit, für die das Instrument vom Versorgungsgerät entkoppelt wird, entlasten, da das Speicherelement betreffende Daten diesem abgegeben werden können. Darüber hinaus führt diese erfindungsgemäße Ausgestaltung auch zu einer wesentlichen Vereinfachung des Versorgungsgeräts, weil beim Vorhandensein mehrerer Versorgungsgeräte, mit denen das Instrument wahlweise kuppelbar ist, es keiner direkten Datenleitung zwischen den Geräten bedarf. Die Datenleitung kann nämlich das Instrument mit seinen jeweils vom Gerät übertragenen Daten übernehmen. Wenn das Instrument mit dem zweiten Gerät gekuppelt wird, kann das zweite Gerät die Daten vom Instrument abrufen, so daß es einer direkten Datenleitung zwischen den Geräten nicht bedarf.

Der Erfindung liegt im weiteren die Aufgabe zugrunde, die Funktion und/oder Steuerung eines Pflegegerätes nach dem Oberbegriff des Anspruches 6 zu verbessern und/oder zu vereinfachen.

Diese Aufgabe wird durch die Merkmale des Anspruches 6 gelöst.

Das Pflegegerät nach Anspruch 6 weist eine Lesevorrichtung zum Auslesen von in dem Instrument gespeicherten Identifizierungs- und/oder Betriebsparameterdaten auf. Hierdurch läßt sich das Pflegegerät in Abhängigkeit von Identifizierungs- und/oder Betriebsparameterdaten steuern. Dabei findet eine selbsttätige Anpassung der Funktion und Steuerung des Pflegegerätes an die jeweils ausgelesenen Identifizierungs- und/oder Betriebsparameter statt. Ferner führt diese Ausgestaltung auch zu einer wesentlichen Vereinfachung des Pflegegerätes, weil es im funktionellen Verbundsystem mit wenigstens einem weiteren Gerät keiner direkten Vernetzung zwischen den Geräten bedarf, da die Datenleitung über das Instrument erfolgen und bei der Kopplung des Instruments mit dem jeweiligen Gerät abgerufen werden kann.

Nachfolgend werden vorteilhafte Ausgestaltungen der Erfindung anhand von bevorzugten Ausführungsbeispielen und Zeichnungen näher erläutert. Es zeigen

Fig. 1 ein erfindungsgemäßes medizinisches oder dentalmedizinisches Benutzungsgerät in schematischer Seitenansicht;

Fig. 2 ein medizinisches oder dentalmedizinisches Instrument als Teil einer Mehrfachausrüstung für das Benutzungsgerät;

Fig. 3 ein erstes Pflegegerät in Form eines Sterilisators in schematischer Seitenansicht, wobei das Pflegegerät nicht Teil des beanspruchten Gegenstand ist;

Fig. 4 das erste Pflegegerät in Form eines Sterilisators in abgewandelter Ausgestaltung in schematischer Seitenansicht;

Fig. 5 ein zweites Pflegegerät in Form eines Reinigungs- und/oder Schmiergerätes in schematischer Seitenansicht;

Fig. 6 ein Ausführungsbeispiel für die in Fig. 1 mit X gekennzeichnete Einzelheit in vergrößerter Darstellung;

Fig. 7 die Einzelheit X in abgewandelter Ausgestaltung;

Fig. 8 die Einzelheit X in weiter abgewandelter Ausgestaltung;

Fig. 9 den Schnitt IX-IX in Fig. 8;

Fig. 10 die Einzelheit X in weiter abgewandelter Ausgestaltung;

Fig. 11 die Einzelheit X in abgewandelter Ausgestaltung in der Seitenansicht;

Fig. 12 ein Instrument mit der Einzelheit X in weiter abgewandelter Ausgestaltung;

Fig. 13 den Schnitt XIII-XIII in Fig. 12;

Fig. 14a und 14b den zeitlichen Verlauf der in dem Ausführungsbeispiel nach den Fig. 12 und 13 erzeugten Spannungen;

Fig. 15 die Einzelheit X in weiter abgewandelter Ausgestaltung;

Fig. 16 die Einzelheit X in weiter abgewandelter Ausgestaltung.

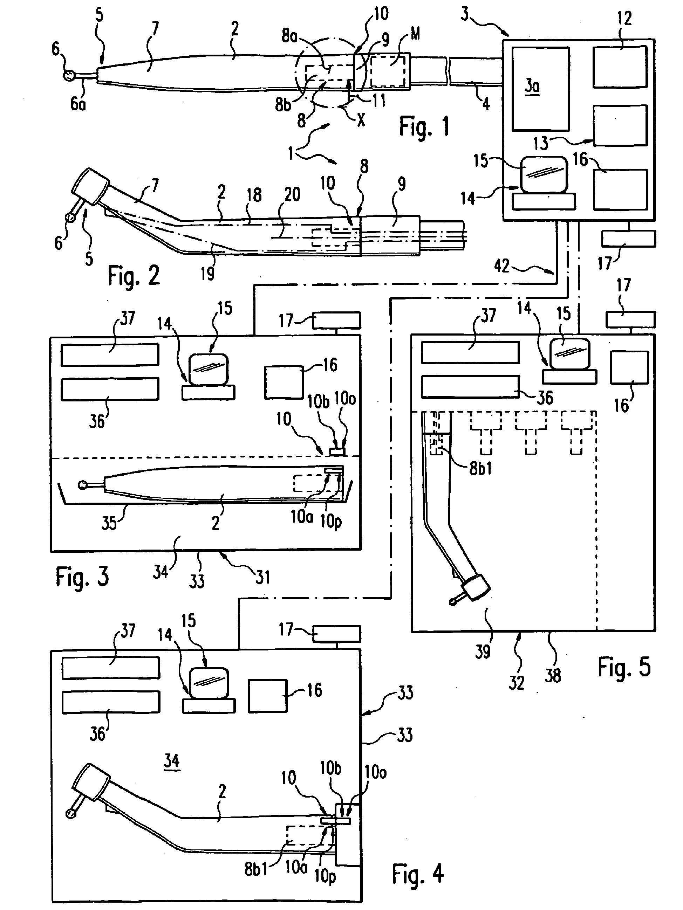

Die Hauptteile der in ihrer Gesamtheit mit 1 bezeichneten Benutzungsvorrichtung bzw. Benutzungsgerätes sind ein Instrument 2 zur Bearbeitung des menschlichen oder tierischen Körpers oder Teilen (Prothesen) davon, ein Versorgungsgerät 3 zum Versorgen des Instruments 2 mit Energie sowie Antriebs- und Benutzungsmedien wie elektrischer Strom, Licht, Wasser und Luft, eine biegsame bzw. flexible Versorgungsleitung 4, die an ihrem hinteren Ende mit dem Versorgungsgerät 3 verbunden ist und an ihrem vorderen freien Ende durch eine lösbare Kupplung mit dem Instrument 2 verbunden ist. Beim Ausführungsbeispiel nach Fig. 1 ist das Instrument 2 ein spanabhebendes Instrument 2, z. B. ein Bohrinstrument, das in seinem vorderen Bereich eine Halte- bzw. Spannvorrichtung 5 aufweist, in die ein Werkzeug 6 mit einem Werkzeugschaft 6a lösbar eingesteckt ist. Das Instrument 2 ist von länglicher bzw. stabförmiger Bauform und erstreckt sich gemäß Fig. 1 beispielsweise gerade. Bei den vorliegenden Ausführungsbeispielen ist das Instrument ein sogenanntes Handstück 7, das durch eine lösbare Kupplung 8, insbesondere eine Steckkupplung oder eine Steck/Drehkupplung, mit einem sogenannten Anschlußteil 9 verbunden ist, das unlösbar oder lösbar, z. B. durch eine Schraubverbindung, mit der Versorgungsleitung 4 verbunden ist. Die Kupplung 8 ist vorzugsweise durch eine Kupplungsausnehmung 8a in dem einen Kupplungsteil und einen mit Bewegungsspiel in die Kupplungsausnehmung 8 passenden Kupplungszapfen 8b am anderen Kupplungsteil gebildet. Vorzugsweise ist die Kupplungsausnehmung 8a im hinteren Endbereich des Handstücks koaxial angeordnet, wobei sie nach hinten ausmündet, und der Kupplungszapfen 8b ragt vom Anschlußteil 9 nach vorne. Die Kupplungsausnehmung 8a und der Kupplungszapfen 8b sind vorzugsweise rund ausgebildet, so daß eine relative Drehbarkeit zwischen diesen Teilen gewährleistet-ist, die die-Handhabung des Instruments 2 bzw. des Handstücks 7 erleichtert. Der Steckkupplung bzw. Steck/Drehkupplung 8 ist eine lösbare Verrastungsvorrichtung 11 zugeordnet, die vorzugsweise manuell überdrückbar ist und somit beim Zusammenstecken selbsttätig in Funktion tritt und durch ein Überdrücken durch eine manuelle Zugausübung außer Funktion bringbar ist. Eine solche Verrastungsvorrichtung 11 ist vorzugsweise durch eine Verrastungskante an dem einen Kupplungsteil und ein Verrastungselement an dem anderen Kupplungsteil gebildet, das gegen eine elastische Rückstellkraft aus einer die Verrastungskante hintergreifenden Kupplungsstellung bewegbar ist. Hierdurch schnappt die Verrastungsvorrichtung 11 beim Zusammenstecken der Kupplungsteile selbsttätig ein, und sie ist zum Lösen wie vorbeschrieben manuell überdrückbar.

Das Versorgungsgerät 3 weist zu seiner Steuerung und Funktion ein Schalttableau 12 zum Einstellen gewünschter Funktion, eine elektronische Steuereinrichtung 13 mit einer Auswerteschaltung zum Auslesen der gelesenen Daten und zum Steuern und Regeln der Funktionen auf, wobei ein Computer 14 zur Steuerung bzw. Regelung integriert sein kann, insbesondere ein PC mit einem Bildschirm 15 zur Anzeige von Betriebsparametern und -daten des Instruments 2 bzw. Werkzeug 6 und des Versorgungsgeräts 3 sowie zugehöriger Funktionen und Zustände, und eine Registriervorrichtung 16 sowie einen Drucker 17 zur Registrierung bzw. Aufzeichnung der vorgenannten Daten oder Informationen.

Zur Ausübung unterschiedlicher Bearbeitungen können der Benutzungsvorrichtung 1 mehrere Instrumente 2 zugeordnet sein, wie es an sich bekannt ist. Fig. 2 zeigt beispielhaft ein Instrument 2 in Form eines sogenannten Winkelstücks. Bei dem oder den Instrumenten 2 kann es sich somit um solche unterschiedlicher Bauweise und Funktion handeln, die bezüglich der Kupplung 8 jeweils passend ausgebildet sind, so daß sie wahlweise an das Anschlußteil 9 ankuppelbar und austauschbar sind. Es kann sich um ein Instrument mit einer sich daran axial erstreckenden Antriebswelle handeln, die durch einen im Anschlußteil 9 angeordneten, z. B. elektrischen Motor M drehbar ist und mit dem Werkzeug 6 in Antriebsverbindung steht. Das Werkzeug 6 kann auch durch eine im vorderen Endbereich des Instruments 2 angeordnete Druckluftturbine antreibbar sein, die durch eine sich vom Versorgungsgerät 3 und längs durch die Versorgungsleitung 4, das Anschlußteil 9 und das Instrument 2 erstreckende Druckluftzuführungsleitung und -abführungsleitung versorgt wird. Zur Zuführung von Benutzungsmitteln bzw. Bearbeitungsmitteln kann das Instrument eine Luftleitung, eine Wasserleitung oder eine Sprayleitung aufweisen, die in an sich bekannter Weise im vorderen Endbereich des Instruments 2 austreten und auf die Bearbeitungsstelle gerichtet sind. Das Instrument 2 kann auch eine Beleuchtungsvorrichtung mit einem sich längs-erstreckenden Lichtleiter oder einer sich längs erstreckenden elektrischen Leitung mit Lampe aufweisen, die sich zu einem im vorderen Endbereich des Instruments 2 angeordneten Lichtaustritt erstreckt. Zur Versorgung der vorbeschriebenen Einrichtungen dienen sogenannte Medienleitungen, die sich von einer Versorgungseinheit 3a des Versorgungsgeräts 3 durch die Versorgungsleitung 4 zum zugehörigen Verbraucher im Anschlußteil 9 oder im Instrument 2 erstrecken. Wenn eine Dreh/Steckkupplung vorgesehen ist, kann eine Medienleitung diese insbesondere koaxial abgedichtet durchsetzen und/oder es können mehrere Medienleitungen die zylindrische Teilungsfuge zwischen dem Kupplungszapfen 8a und der Kupplungsausnehmung 8b Z-förmig abgedichtet durchsetzen, wie es an sich bekannt ist. Zur Verdeutlichung dieser an sich bekannten Sachverhalte sind in Fig. 1 beispielhaft drei Medienleitungen 18, 19, 20 für Wasser, Druckluft und Licht andeutungsweise dargestellt, die die Kupplung 8 koaxial bzw. Z-förmig durchsetzen.

Zur Einstellung unterschiedlicher Benutzungs- bzw. Betriebsprogramme weist die Benutzungsvorrichtung 1 wenigstens zwei Programmebenen auf. In einer ersten Programmebene kann der Benutzer zwischen einer Vielzahl von hersteller- bzw. werkseitig vorgegebenen Behandlungs- bzw. Betriebsprogrammen auswählen. In einer zweiten Programmebene kann der Benutzer zwischen einer Vielzahl von benutzerspezifisch erstellten Betriebsprogrammen auswählen. Nach Auswahl eines der Betriebsprogramme steuert die Steuereinrichtung 13 das Instrument 2 bzw. das Werkzeug 6 abhängig von den dem ausgewählten Betriebsprogramm entsprechenden Betriebsparameterinformationen bzw. -daten. Der Benutzer kann beliebig zwischen den benutzerspezifisch abgespeicherten Betriebsprogrammen und den herstellerseitig vorgegebenen Betriebsprogrammen wählen.

Die jedem Betriebsprogramm zugeordneten Betriebsparameterdaten enthalten Informationen über ein auszuwählendes Instrument 2 und dessen betriebs- und/oder benutzungsspezifische Daten, z. B. Drehzahl, Untersetzungsverhältnis, Wirkungsgrad, Benutzungsmittel sowie Benutzungsmittelmenge usw.

Die Registriervorrichtung 16 ermöglicht es, gewünschte betriebs- und benutzerspezifische Daten und Informationen über die jeweilige Betriebszeit hinaus zu registrieren und abzurufen, z. B. zwecks Anzeige am Bildschirm 15 oder durch Ausdruck mit dem Drucker 17.

Zur Identifizierung der Instrumente 2 weisen diese im Bereich des Kupplungsteils des Instruments 2 ein Codierung 10a auf, die von einer Lesevorrichtung 10b lesbar ist, die im Bereich des anderen Kupplungsteils am Anschlußteil 9 bzw. an der Versorgungsleitung 4 angeordnet ist und durch wenigstens eine zugehörige Datenleitung mit der Steuereinrichtung 13 verbunden ist.

Die Registriervorrichtung 16 ist vorzugsweise dazu eingerichtet, bei solchen Daten, die im Funktionsbetrieb variabel sein können, nicht nur den z. B. vom Benutzer eingestellten Sollwert, sondern auch den tatsächlich erreichten Istwert zu registrieren. Hierbei kann es sich z. B. um eine Maximaldrehzahl handeln, die je nach Belastung und Leistung unterschiedlich sein kann. Die Registrierung des Istwertes ermöglicht es, den tatsächlichen Sachverhalt festzuhalten, abzurufen und darzustellen, um z. B. die Benutzung auch nachträglich verdeutlichen und beurteilen zu können.

Die mit 10 bezeichnete Identifikationsvorrichtung kann z. B. optisch, elektromagnetisch oder induktiv wirksam sein. Bei einer Steck/Drehkupplung 8 mit einer Kupplungsausnehmung 8a und einem Kupplungszapfen 8b ist es vorteilhaft, die Identifikationsvorrichtung zu beiden Seiten der zylindrischen oder radialen Teilungsfuge der Kupplung 8 anzuordnen. Die Codierung 10a kann beim Ausführungsbeispiel in oder an der die Kupplungsausnehmung 8a umgebenden Hülsenwand 2a des Instruments 2 angeordnet sein, wobei die Lesevorrichtung 10b im Kupplungszapfen 8b oder der Hülsenwand 2a gegenüberliegend im Anschlußteil 9 angeordnet sein kann. Im ersteren Fall ist die Wirkrichtung der Identifikationsvorrichtung 10 radial, wobei die Daten der Lesevorrichtung 10b von der Mantelfläche des Kupplungszapfens 8b her zugänglich sind, während im zweiten Fall die Lesevorrichtung 10b im axialen Projektionsbereich der Hülsenwand 2a angeordnet und mit den Daten von der Ringstufenfläche am Anschlußteil 9 her zugänglich ist.

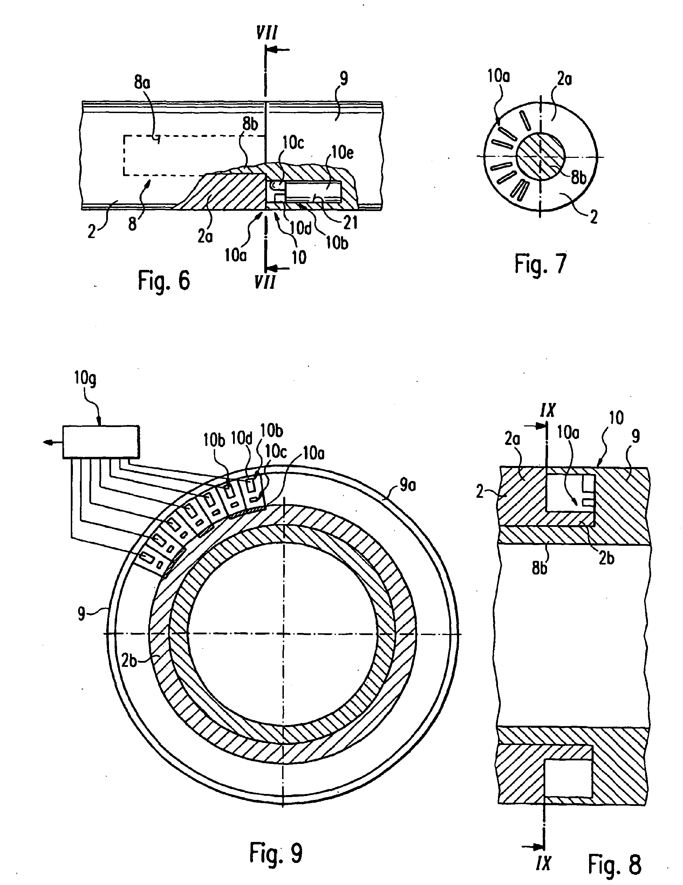

Beim Ausführungsbeispiel nach Fig. 6, bei dem gleiche oder vergleichbare Teile mit gleichen Bezugszeichen versehen sind, ist eine optische Identifikcationsvorrichtung 10 mit einer Codierung 10a, z. B. in Form eines Mustercodes, insbesondere eines Strichcodes, an einer Rückenfläche, hier die Rückenfläche der Hülsenwand 2a, und einer Lesevorrichtung 10b hinter der radialen Teilungsfuge im Anschlußteil 9. Diese Lesevorrichtung 10b weist eine z. B. durch eine Leuchtdiode gebildete Lichtquelle 10c und einen Photozellen-Sensor 10d auf, die vorzugsweise in einem gemeinsamen, insbesondere zylindrischen Träger 10e angeordnet bzw. eingebettet sind, der in einer Ausnehmung 21 fest eingesetzt ist, die an der Ringstufe der radialen Teilungsfläche mündet. Der Mustercode (Hell- und Dunkelflächen) ist vorzugsweise durch radiale Striche gebildet, zwischen denen unterschiedliche Winkel eingeschlossen sind, wie es Fig. 7 zeigt. Die Leserichtung verläuft axial bzw. achsparallel.

Beim Ausführungsbeispiel nach Fig. 8 und 9, bei dem gleiche oder vergleichbare Teile mit gleichen Bezugszeichen versehen sind, ist Die Leserichtung der Lesevorrichtung 10b radial gerichtet, hier radial nach innen. Beim Ausführungsbeispiel sind mehrere Photozellensensoren 10d bezüglich der Codierung 10a in Umfangsrichtung nebeneinander und radial außen angeordnet. Die Codierung 10a kann durch eine Hell-Dunkelmarkierung auf einer äußeren Umfangs- oder Segmentfläche gebildet sein, insbesondere durch einen Strichcode. Wie Fig. 9 zeigt, sind die Codierung 10a und die Lesevorrichtung 10b an einander radial gegenüberliegenden Teilen der Kupplungsteile angeordnet. Hierbei kann die Codierung 10a an der Außenmantelfläche eines inneren Rücksprunges 2b am Instrument 2 angeordnet sein, der von der Lesevorrichtung 10b übergriffen ist. Die vorzugsweise in einer Mehrzahl vorhandenen Photozellen können zum einen an einer nach vorne gerichteten Stirnfläche des Anschlußteils 9 angeordnet und zum anderen in ein Sensorringsegment zusammengefaßt sein. Ein die Lesevorrichtungen 10b aufnehmender Freiraum kann durch einen vorspringenden Hülsenansatz 9a des Anschlußteils 9 abgedeckt sein. Ein Parallel-Seriell-Umsetzer oder eine Auswerteschaltung ist mit 10g bezeichnet.

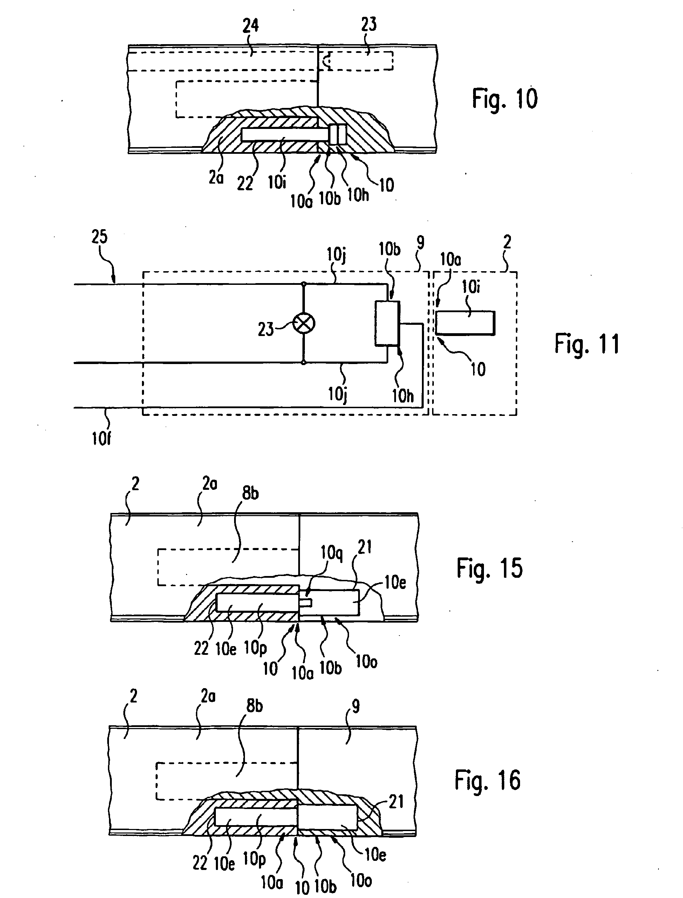

Das Ausführungsbeispiel nach Fig. 10 und 11 zeigt eine Identifikationsvorrichtung 10 mit einem insbesondere magnetischen Sensor 10h, z. B. ein Hallsensor oder eine Feldplatte, in entsprechender Anordnung hinter der radialen Teilungsfuge des Anschlußteils 9. Dem Sensor 10h ist in der Hülsenwand 2a ein Magnet 10i gegenüberliegend in einer rückseitig offenen Ausnehmung 22 angeordnet ist. Fig. 11 zeigt schematisch die Anordnung und den elektrischen Anschluß des Sensors 10h beim Vorhandensein einer Beleuchtungseinrichtung mit einer Lampe 23, die beim Ausführungsbeispiel bezüglich des Sensors 10h in Umfangsrichtung versetzt im Anschlußteil 9 angeordnet ist und Licht in einen der Lampe 23 gegenüberliegend im Instrument 2 angeordneten und sich in der Hülsenwand 2a nach vorne erstreckenden Lichtleiter 24 einspeist. Der Sensor 10h ist durch elektrische Leitungsabschnitte 10j an den Stromkreislauf 25 der Lampe 23 angeschlossen. Die Codierung 10a kann bei diesem Ausführungsbeispiel dadurch gebildet sein, daß unterschiedliche Instrumente 2 Magnete 10i mit unterschiedlich starken elektromagnetischen Feldern aufweisen und/oder die Luftspaltabstände zwischen dem Hallsensor 10h und den Magneten 10i jeweils im gekuppelten Zustand unterschiedlich sind. Hierdurch werden im Sensor 10h unterschiedliche elektrische Spannungen erzeugt, die durch eine sich vom Sensor 10h durch das Anschlußteil 9 und die Versorgungsleitung 4 nach hinten zur elektronischen Steuereinrichtung 13 erstreckenden Signalleitung der elektronischen Steuereinrichtung 13 zur Einstellung der gewünschten Betriebsdaten zugeführt werden.

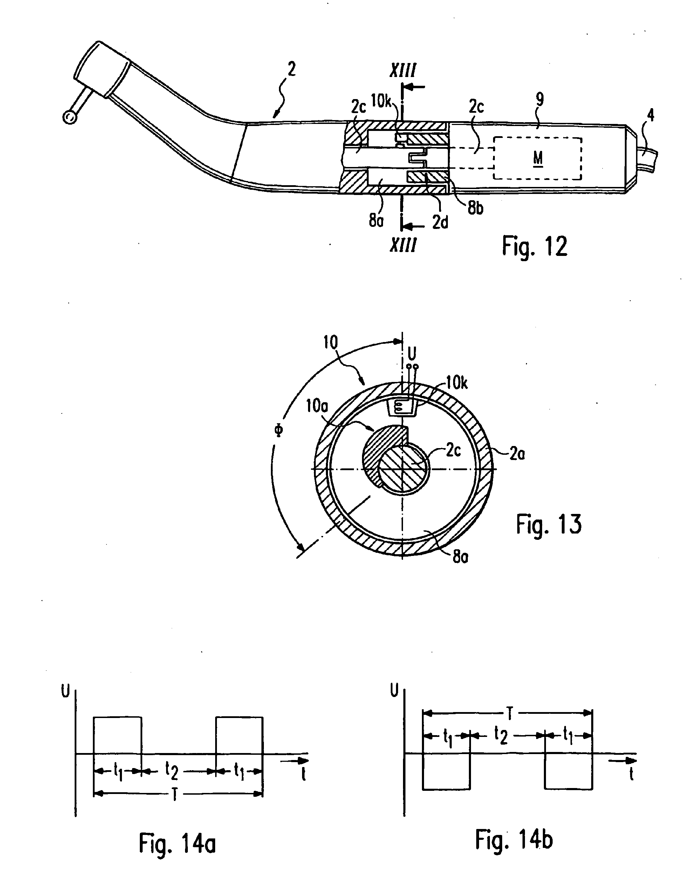

Beim Ausführungsbeispiel nach Fig. 12 bis 14 ist die Identifikationsvorrichtung 10 für ein Instrument 2 eingerichtet, in dem im Bereich der Kupplung 8 ein Drehteil gelagert ist, bei dem es sich beim Ausführungsbeispiel um eine Antriebswelle 2c handelt, die in einem Antriebskanal des Instruments 2 drehbar gelagert ist. Das hintere Ende der Antriebswelle 2c ist durch eine Steckkupplung 2d mit einem im Anschlußteil 9 drehbar gelagerten Antriebswellenabschnitt 9a gekuppelt, bei dem es sich z.B. um die Antriebswelle des Motors M handeln kann. Bei dieser Ausgestaltung ist ein Sensor 10k vorgesehen, der am Anschlußteil 9 gehalten ist, und zwar an der Stirnseite des Kupplungszapfens 8b. Auf der Antriebswelle 2c des Instrumentes 2 befindet sich ein Geberelement 10a aus ferromagnetischem Material, das sich über einen Winkel Φ erstreckt und eine über den Winkel Φ abnehmende Höhe hat. Es hat - in der Abwicklung gesehen - die Form einer Rampe. Das Geberelement 10a passiert bei Drehung der Antriebswelle 2c den Sensor 10k. Der Sensor 10k ist z.B. eine Spule, in der das sich vorbeibewegende Geberelement 10a eine von der Drehgeschwindigkeit und der Drehrichtung der Antriebswelle 2c abhängige Spannung U erzeugt.

Es ist nun eine Codierung der Instrumente 2 durch Wahl der Anfangs- und Endehöhe des Geberelementes 10a und/oder durch Wahl des Winkels Φ, über den sich das Geberelement erstreckt, möglich. Mit anderen Worten, eine Codierung ist durch die Wahl einer bestimmten Rampenform möglich.

In den Fig. 14a und 14b sind die Ausgangssignale des Sensorelementes 10k dargestellt, und zwar als Spannung U in Abhängigkeit von der Zeit. Bei Drehung der Antriebswelle 2c in Fig. 13 entstehen Spannungsimpulse.

In Fig. 14a sind die Spannungsimpulse bei Rechtsdrehung der Antriebswelle 2c gezeigt. In diesem Fall hat die Rampe in Bezug auf den Sensor 10k eine ansteigende Form, mit der Folge, daß positive Spannungsimpulse erzeugt werden.

In Fig. 14b sind die Spannungsimpulse bei Linksdrehung der Antriebswelle gezeigt. Da die Rampe hier in Bezug auf den Sensor 10k eine abfallende Form hat, sind die Spannungsimpulse negativ.

Die Impulse haben in beiden Figuren eine zeitliche Dauer $t_1$ und einen zeitlichen Abstand $t_2$. Die Periodendauer beträgt T. Der Zusammenhang zwischen dem in Fig. 13 gezeigten Winkel Φ und den in den Fig. 14a und 14b angegebenen Zeiten ergibt sich aus der Formel:

$$\Phi = 360\,° \times t_1/T$$

Die Drehzahl n der Antriebswelle 2c ist n = 1/T. Durch Messung von $t_1$ und $t_2$ bzw. T kann auf den Codierungswinkel Φ geschlossen werden.

Durch Messung der Amplitude der Spannung U kann bei Kenntnis der Drehzahl n auf die Rampenform geschlossen werden, die damit auch zur Codierung herangezogen werden kann.

Die Polarität der Spannungsimpulse gibt Auskunft über den Drehsinn (links oder rechts rum) der Antriebswelle 2c.

Bei den Ausführungsbeispielen nach Fig. 15 und 16 ist jeweils die Codierung 10a veränderlich. Die Codierung 10a ist nicht nur in der Lage, bestimmte Daten bereitzustellen, die von der Lesevorrichtung 10b lesbar sind, sondern die Codierung 10a läßt sich bezüglich der in ihr gespeicherten Daten verändern und zwar mit Daten, die ihr von der elektronischen Steuereinrichtung 13 übertragen werden. Hierbei kann es sich um herstellerseitig vorgegebene oder vom Benutzer erstellte Betriebsdaten, z.B. wenigstens ein Betriebsprogramm, handeln. Es kann sich auch um andere Informationen handeln, z. B. die den Betrieb des Instruments 2 und/oder den zu behandelnden Patienten und/oder die Behandlung des Instruments 2 im Sinne von Pflegemaßnahmen betreffen. Hierbei ist die Lesevorrichtung 10b zugleich Datensender und -empfänger 10o und die Codierung 10a ist durch eine Übertragung von Daten vom Datensender 10o veränderlich und vorzugsweise durch ein Speicherelement 10p zum Überschreiben seiner Daten durch die vom Datensender 10o übertragenen Daten ausgebildet. Fig. 15 und 16 zeigen beispielhaft zwei Ausführungsbeispiele für eine solche Datenübertragungseinrichtung, bei der die Daten wahlweise sowohl in die eine als auch in die andere Richtung zwischen der elektronischen Steuereinrichtung 13 und dem Speicherelement 10p übertragen, d. h. abgerufen und wieder eingelesen werden können.

Fig. 15 zeigt ein Ausführungsbeispiel mit einer mechanischen Kontaktierung 10q zwischen dem Datenempfänger und -sender 10o und dem Speicherelement 10p. Die einzelnen elektrischen Kontakte sind aus Vereinfachungsgründen nicht dargestellt. Fig. 16 zeigt eine berührungslose Datenübertragung zwischen dem Datenempfänger und -sender 10o und dem Speicherelement 10p. Die Datenübertragung kann z.B. elektromagnetisch oder induktiv erfolgen. Das Speicherelement 10p kann durch einen Transponder gebildet sein, nämlich ein Empfängs-Sendegeräte, das nach dem Abfrage-Antwort-System arbeitet. Bei dem Datenempfänger und -sender 10o kann es sich um einen korrespondierenden Transponder handeln. In beiden Fällen handelt es sich um Speicherelemente, die jeweils durch ein vorgefertigtes, insbesondere stiftförmiges Bauteil gebildet und in einer den Ausnehmungen 21,22 entsprechenden Ausnehmung eingesetzt sind.

Aufgrund der Übertragung von solchen Daten auf das Instrument 2, die für eine beabsichtigte Benutzung des Instruments 2 am Patienten und/oder für eine Behandlung des Instruments 2 selbst, z.B. Pflegemaßnahmen, und/oder für eine Registrierung von Daten bestimmend sind, ist es möglich, das Instrument 2 mit einem anderen Gerät zu kombinieren, das einen Datenempfänger bzw. eine Lesevorrichtung aufweist, die diese Daten empfangen und vorzugsweise Daten auf das Speicherelement 10p rückübertragen kann. Bei einem solchen weiteren Gerät kann es sich z.B. um eine zweite, den Benutzer zur Verfügung stehende Benutzungsvorrichtung (nicht dargestellt) und/oder ein Pflegegerät handeln, was noch beschrieben wird. Dabei bedarf es bei den Ausführungsbeispielen nach Fig. 15 und 16 keiner Datenleitung direkt zwischen dem Versorgungsgerät 3 und dem weiteren Gerät, denn die Datenleitung kann durch das Instrument 2 erfolgen.

Bei allen vorbeschriebenen Ausführungsbeispielen ist es vorteilhaft, die empfindlichen Bauteile, hier die Lesevorrichtung 10b am Anschlußteil 9 oder am von diesem vorragenden Kupplungszapfen 8b anzuordnen und die Codierung am Instrument 2 anzuordnen. Hierdurch sind die weniger empfindlichen Teile der Identifikationsvorrichtung 10 am Instrument 2 angeordnet, das bei einer Reinigung, Desinfektion und/oder Sterilisation beträchtlichen Beanspruchungen, insbesondere Temperaturen unterliegt. Außerdem ist die elektronische Steuereinrichtung 13 durch wenigstens eine geeignete Datenleitung mit der Lesevorrichtung 10b bzw. mit dem Datenempfänger und -sender 10o verbunden.

Die Fig. 3 bis 5 zeigen jeweils beispielhaft ein solches zweites Gerät als Pflegegerät, wobei Fig. 3 und 4 jeweils ein Sterilisiergerät 31 und Fig. 5 ein Pflegegerät 32 zum Reinigen und Ölen zeigen.

Das Sterilisiergerät 31 nach Fig. 3 weist in einem Gehäuse 33 eine Sterilisierkammer 34 auf, die durch eine nicht dargestellte Tür zu öffnen und zu schließen ist, so daß ein zu sterilisierendes Instrument 2 in die Sterilisierkammer 34 eingebracht und abgelegt werden kann, z.B. auf eine Ablage oder Schale 35. Um mit Instrumenten 2 gemäß den vorbeschriebenen Ausgestaltungen nach Fig. 6 bis 11 und Fig. 15 und 16 benutzt werden zu können, ist dem Sterilisiergerät 31 eine Lesevorrichtung 10b oder ein Datenempfänger und -sender 10o zugeordnet, der sich in oder außerhalb der Sterilisierkammer 34 befmden kann und das Instrument 2 anhand der Codierung 10a identifizieren kann und die für die Desinfizierung erforderlichen Versorgungsmaßnahmen durchführt, wozu z.B. das Erwärmen der Sterilisierkammer 34 auf eine erforderliche Sterilisierungstemperatur, z.B wenigstens 135°, und ggf. auch das Erzeugen eines Über- oder Unterdrucks in der Sterilisierkammer 34 umfassen. Mit den Bezugszahlen 36 und 37 sind andeutungsweise ein Versorgungsgerät zum Bereitstellen der Sterilisiermedien und eine elektronische Steuereinrichtung mit einer Auswerteschaltung für die gelesenen Daten und zum Steuern der Sterilisiermaßnahmen zugeordnet. Die von der Lesevorrichtung 10b bzw. vom Datenempfänger 10o gelesenen Daten werden der Steuereinrichtung 37 zwecks Erkennung des Instruments 2 und Steuerung der Sterilisiermaßnahmen zugeführt.

Das Ausführungsbeispiel gemäß Fig. 4 unterscheidet sich von dem gemäß Fig. 3 dadurch, daß in der Sterilisierkammer 34 ein dem Kupplungszapfen 8b nach Form und Größe entsprechender Kupplungszapfen 8b1 angeordnet ist, z.B. an einer Wand der Sterilisierkammer 34 befestigt ist, auf den das Instrument 2 mit seiner Kupplungsausnehmung 8a aufsteckbar ist. Auch bei diesem Ausführungsbeispiel kann die Kupplung 8 entsprechend umgekehrt ausgebildet sein, d.h. es kann im Sterilisierraum 34 eine Kupplungsausnehmung angeordnet sein, in die ein Instrument 2 mit einem abstehenden Kupplungszapfen einsteckbar ist. Hierdurch ist in der Sterilisierkammer 34 eine Positioniervorrichtung für das Instrument 2 geschaffen, die die Positionierung und Erkennung des Instruments 2 und der Daten erleichtert.

Das Pflegegerät 32 gemäß Fig. 5 weist in einem Gehäuse 38 eine Pflegekammer 39 auf, die durch eine nicht dargestellte Tür wahlweise zu öffnen und zu schließen ist, und in die für einen Pflegevorgang das Instrument 2 in die Pflegekammer 39 eingebracht und positioniert wird, z. B. auf eine Ablage oder Schale oder an einem Kupplungsteil, insbesondere an einem Kupplungszapfen 8b1, wie es für das Sterilisiergerät 31 bereits beschrieben worden ist. Das Pflegegerät 32 kann für unterschiedliche Instrumente 2 mehrere Kupplungsteile bzw. Kupplungszapfen 8b1 aufweisen, wie es in Fig. 5 andeutungsweise dargestellt ist. Auch die Sterilisiergeräte 31 gemäß Fig. 3 und 4 können mehrere solcher Kupplungsteile für unterschiedliche Instrumente 2 aufweisen. Auch das Pflegegerät 32 weist ein Versorgungsgerät 36 und eine elektronische Steuereinrichtung 37 mit einer Auswerteschaltung für die gelesenen Daten zum Bereitstellen von Pflegemedien, z.B. Druckluft oder ein Pflegemittel, insbesondere Öl, und Zuführen dieser Medien zum Instrument 2 und zum Steuern dieser Maßnahmen zwecks Durchführung der Reinigung und/oder Pflege. Das Pflegegerät 32 kann im Übrigen von der eingangs beschriebenen Art sein.

Wie bereits das Pflegegerät 31 weist auch das Pflegegerät 32 jeweils eine den zugehörigen Kupplungsteilen 8b1 zugeordnete Lesevorrichtung 10b oder einen Datenempfänger und sender 10o auf, die bzw. der das eingebrachte bzw. angekuppelte Instrument 2 anhand einer Codierung 10a bzw. Datenspeicherung erkennt und die Daten an die Steuereinrichtung 37 zur Steuerung des Versorgungsgeräts 36 und zur Ausführung der Pflegemaßnahmen weiterleitet.

Im ersten oder zweiten Pflegegerät, hier dem Sterilisiergerät 31 und dem Pflegegerät 32 oder einem zusätzlichen oder anderen Gerät kann jeweils ein Computer 14a gegebenenfalls mit Bildschirm 15 zur Verarbeitung der jeweiligen Daten und zur Steuerung entsprechend den Daten zugeordnet sein. Außerdem kann jedem dieser Geräte eine Registriervorrichtung 16 zur Registrierung von Betriebsdaten und Informationen z. B. zur Registrierung der durchgeführten Pflegemaßnahmen, insbesondere der Sollwerte und Istwerte dieser Betriebsdaten zugeordnet sein. Hierbei kann es sich bei dem Sterilisiergerät 31 z.B. um die Sterilisierzeit und/oder die Sterilisiertemperatur und/oder den Sterilisierüberdruck oder - unterdruck handeln. Beim Pflegegerät 32 kann es sich z.B. um die Pflege- bzw. Funktionszeit von Pflegemaßnahmen wie Durchblaszeit und/oder Ölungszeit und/oder Ölmenge handeln.

Beim Ausführungsbeispiel sind vereinfacht dargestellte Zuführungsleitungen 41 für die Pflegemedien, hier Druckluft und Öl, vorhanden, die sich von einem jeweils zugehörigen Vorrat zu dem oder den Kupplungsteilen 8b1 erstrecken und im angekuppelten Zustand des Instruments 2 mit den Medienkanälen des Instruments 2, hier den Kanälen 18, 19 und/oder dem einen sog. Antriebskanal, in dem die Antriebswelle des Instruments 2 drehbar gelagert ist, in Verbindung steht zwecks Reinigung durch ein Durchblasen von Druckluft und Pflege des Antriebskanals durch Einführung bzw. Einblasung von Pflegemittel, insbesondere Öl.

Bei einer Ausgestaltung gemäß Fig. 15 und 16 kann auf eine direkte Vernetzung der Geräte 1 und/oder 31 und/oder 32 durch Datenleitungen 42 verzichtet werden, da die erforderlichen Daten im Instrument 2 gespeichert werden können und mit dem Instrument 2 an das jeweilige Gerät übertragen bzw. vom jeweiligen Gerät abgerufen werden können. Bei den Ausgestaltungen, bei denen die Codierung 10a mittels der Lesevorrichtung 10b nur gelesen werden kann, ist eine Vernetzung 42 der wenigstens zwei Geräte erforderlich. Die Vernetzung kann mittels besonderer elektrischer Leitungen oder kabellos erfolgen, beispielsweise über Infrarot- oder Funkübertragungsstrecken. Es ist möglich, den wenigstens zwei Geräten 1 und/oder 31 und/oder 32 und/oder wenigstens einem weiteren Gerät, z. B. ein zweites Benutzungsgerät, mit einer gemeinsamen elektronischen Steuereinrichtung 13 bzw. mit einem gemeinsamen Computer 14, einer gemeinsamen Registriervorrichtung 16 und einem gemeinsamen Drucker 17 auszurüsten. Bei einer solchen Ausgestaltung ist eine Vernetzung 42 auch dann erforderlich, wenn Instrumente 2 gemäß Fig. 15 und 16 zu pflegen bzw. zu behandeln sind, da die Steuerung gemeinsame erfolgt.

Nachfolgend werden diverse Vorteile und Funktionen der erfindungsgemäßen Ausgestaltungen beschrieben.

Beim Ausführungsbeispiel nach Fig. 1 und 2 bildet das Benutzungsgerät 1 mit einem gegebenenfalls vorhandenen Behandlungsstuhl einen Behandlungsplatz, wobei es sich um ein Chirurgiegerät, insbesondere ein Mikrochirurgiegerät, oder um ein dentales Behandlungsgerät handeln kann. Aufgrund der Registrierung nicht nur der Sollwerte, sondern auch der Istwerte von Betriebsdaten, z. B. bei der Benutzung aufgetretene maximale Drehmomente oder maximale Werkzeugdrehzahlen, und weitere Betriebsinformationen, wie z. B. die Uhrzeit oder das Datum, lassen sich nicht nur während der Bearbeitung sondern auch nach der Bearbeitung abrufen und vorübergehend oder bleibend darstellen, z. B. am Bildschirm 15 und durch Ausdrucken am Drucker 17. Es kann somit die Benutzung des Instruments 2 bei der Bearbeitung des Patienten oder Körpers und/oder die Behandlung des Instruments 2 selbst, z. B. zum Zweck der Pflege, nachgewiesen und beurteilt werden, was insbesondere im Hinblick auf Garantieansprüche und Lebensdauer von Bedeutung ist.

Diese Vorteile und Maßnahmen gelten nicht nur für ein Behandlungsgerät nach Fig. 1 und 2 sondern auch für ein Pflegegerät 32 zur Durchführung von Reinigungs- und/oder Desinfizier- und/oder Sterilisiermaßnahmen.

Die erfindungsgemäßen Ausgestaltungen ermöglichen das Erkennen und Erfüllen folgender Anforderungen des bzw. der Instrumente 2.

a) Um welches Instrument 2 handelt es sich?
b) Welche Anforderungen stellt das Instrument 2 ? Z. B. Betriebsdaten wie Drehzahl, Drehmoment, welches Benutzungsmedium (Luft, Wasser, elektrischer Strom, usw. ist erforderlich?

c) In welchem Zustand befindet sich das Instrument ? Z. B. wieviele Betriebsstunden hat das Instrument bereits gelaufen, ist das Instrument 2 gereinigt oder desinfiziert oder sterilisiert oder gepflegt?

Die Anforderungen a) und b) erkennt die jeweils einzelne oder gemeinsame elektronische Steuereinrichtung durch Vergleich und Auswertung der erkannten Identifikationsdaten mit in der elektronischen Steuereinrichtung gespeicherten Daten. Die Anforderung c) wird dadurch erfüllt, daß die betreffenden Betriebsdaten ermittelt und registriert werden und somit die betreffenden Registraturdaten den jeweiligen Zustand des Instruments 2 repräsentieren. Durch eine fortlaufende Registrierung von Daten läßt sich auch der Betriebsablauf registrieren und somit nicht nur bestimmte Betriebsfunktionen der Benutzung und/oder der Pflege registrieren. Es lassen sich auch bestimmte Betriebsabläufe oder Daten so in Abhängigkeit von vorherigen Betriebsabläufen oder gespeicherten Daten steuern, daß ein bestimmter Betriebsablauf nur dann erfolgt, wenn ein vorheriger Betriebsablauf stattgefunden hat oder ein bestimmter Betriebszustand erreicht wird, der durch Daten registriert oder gespeichert ist. Wenn z. B. ein Instrument 2 nicht gepflegt worden ist, erkennt die Steuereinrichtung diesen Mangel aufgrund des Fehlens betreffender Daten, und sie sperrt den nachfolgenden Betriebsablauf oder zeigt diesen Mangel durch ein Warnsignal an oder registriert diesen Mangel. Wenn z. B. ein Instrument 2 nicht gepflegt worden ist oder unzureichend gepflegt worden ist, wenn z. B. die Sterilisiertemperatur und/oder die Sterilisierzeit nicht dem Sollwert entspricht, erkennt dies die Steuereinrichtung spätestens beim Ankoppeln des Instruments an das Versorgungsgerät 3, wobei die Steuereinrichtung die Benutzung sperren kann oder zumindest den Mangel anzeigen kann.

Eine solche Sperrung oder Anzeige läßt sich auch dann steuern, wenn ein Instrument 2 eine vorgegebene Anzahl Betriebsstunden überschreitet, die ebenfalls durch Registrierung zugehöriger Daten überwacht werden können.

Der Erfindung liegt die Erkenntnis zugrunde, daß Betriebsabläufe und Zustände von wenigstens einem Instrument 2 an wenigstens zwei Benutzungs- und/oder Versorgungsgeräten erkannt werden können, wenn die Geräte und/oder die Instrumente mit entsprechenden Erkennungsdaten gespeichert sind. Ein Instrument 2 wird z. B. mit einem Pflegegerät 31 oder 32 zu seiner Pflege gekoppelt. Dieses Gerät erkennt die jeweilige Art des Instruments. Das Gerät ruft nun die gespeicherten Daten für genau dieses Instrument 2 ab. Nach diesen Vorgaben (Ölmenge, Ausblaszeit) startet der Pflegezyklus. Gespeichert werden nach abgeschlossener Pflege zugehörige Durchlauf-oder Pflegedaten, wozu auch die Seriennummer des Instruments 2 dienen kann.

Wenn auch wenigstens ein anderes Gerät in der Lage ist, die Daten des vorherigen Betriebsablaufs abzurufen und zu speichern, kann man durch die Datenvernetzung der Geräte Betriebsabläufe mehrerer Geräte nachverfolgen, z. B. die Pflege und die Benutzung des Instruments 2.

Z. B. Instrument A wurde am 16.01.2001 um 10.00 Uhr im Pflegegerät 32 gepflegt. Instrument A wurde am 16.01.2001 um 10.05 Uhr im Sterilisiergerät 31 sterilisiert. Instrument A wurde am 16.01.2001 um 11.00 Uhr am Benutzungsgerät 1 (Patient 4) insgesamt fünf Minuten betrieben. Dadurch kann nicht nur der Betriebsablauf festgehalten werden, sondern es können auch Fehler festgestellt werden, z. B. durch eine Fehlermeldung, die die zugehörige Steuereinrichtung errechnet oder beim Koppeln des nicht sterilisierten Instruments 2 erkennt.

## Patentansprüche

1. Medizinisches oder dentalmedizinisches Instrument (2) mit einem Speicherelement (10p) für Identifizierungs- und/oder Betriebsparameterdaten, wobei das Speicherelement (10p) zum Überschreiben der Daten durch andere Daten ausgebildet ist, und wobei das Instrument (2) ein Kupplungsteil aufweist, mit dem es an ein korrespondierendes Kupplungsteil einer Versorgungsleitung (4) eines Versorgungsgerätes (3) zum Versorgen des Instruments (2) mit Medien ankuppelbar ist, wobei das Kupplungsteil durch eine hinten am Instrument (2) angeordnete Kupplungsausnehmung (8a) oder einen vom Instrument hinten abstehenden Kupplungszapfen (8b) gebildet ist, **dadurch gekennzeichnet, daß** das Speicherelement (10p) in der die Kupplungsausnehmung (8a) umgebenden Hülsenwand (2a) angeordnet ist und für das korrespondierende Kupplungsteil von der Innenmantelfläche der Hülsenwand (2a) oder von der hinteren Ringstirnfläche der Hülsenwand (2a) her zugänglich ist oder im Kupplungszapfen (8b) angeordnet ist und von der Mantelfläche des Kupplungszapfens (8b) her zugänglich ist oder in der Kupplungszapfenbasis hinter einer ringförmigen Stufenfläche angeordnet ist und von der ringförmigen Stufenfläche her zugänglich ist.

2. Instrument nach Anspruch 1, **dadurch gekennzeichnet, daß** das Speicherelement (10p) Kontaktmittel (10q) zur Aufnahme und/oder Abgabe von Daten oder Mittel zur kontaktlosen Aufnahme und/oder Abgabe von Daten aufweist.

3. Verwendung eines Instruments (2) nach Anspruch 1 oder 2, wobei dieses mindestens eine Benutzungsphase und mindestens eine Pflegephase durchlaufen soll,

wobei

in das Speicherelement (10p) mindestens ein Parameter aus der Benutzungsphase eingeschrieben und zur Steuerung der Pflegephase ausgelesen wird,

und/oder in das Speicherelement (10p) mindestens ein Parameter aus der Pflegephase eingeschrieben und zur Steuerung der Benutzungsphase ausgelesen wird.

4. Versorgungsgerät (3) zum Versorgen wenigstens eines medizinischen oder dentalmedizinischen Instruments (2) nach einem der Ansprüche 1 bis 2 mit Medien,

wobei das Versorgungsgerät (3) computergesteuert ist und sich vom Versorgungsgerät (3) eine Versorgungsleitung (4) erstreckt, an deren Ende ein Kupplungsteil angeordnet ist, mit dem die Versorgungsleitung (4) mit dem Instrument (2) verbindbar ist, und wobei im Bereich des Kupplungsteils ein Datenempfänger angeordnet ist zum Empfangen von Identifizierungs- und/oder Betriebsdaten, die in einem Speicherelement (10p) des Instruments (2) gespeichert sind,

**dadurch gekennzeichnet,**

**daß** das Kupplungsteil durch einen von der Versorgungsleitung (4) vorstehenden Kupplungszapfen (8b) oder eine Kupplungsausnehmung gebildet ist und der Datenempfänger im Kupplungszapfen (8b) oder hinter der Ringschulterfläche, von der der Kupplungszapfen sich erstreckt oder in einer die Kupplungsausnehmung umgebenden Hülsenwand angeordnet ist.

5. Versorgungsgerät nach Anspruch 4,
**dadurch gekennzeichnet,**
**daß** der Datenempfänger auch ein Datensender (10o) zum Übertragen von Daten auf das Speicherelement (10p) ist.

6. Pflegegerät (31; 32) für mindestens ein medizinisches oder dentalmedizinisches Instrument (2) nach einem der Ansprüche 1 bis 3,
**dadurch gekennzeichnet,**
**daß** es ein Kupplungsteil aufweiset, durch das das Instrument (2) mit dem Pflegegerät (31; 32) kuppelbar ist,
wobei das Kupplungsteil durch einen vorstehenden Kupplungszapfen (8b1) oder eine Kupplungsausnehmung gebildet ist und im Kupplungszapfen (8b1) oder hinter der Ringschulterfläche, von der der Kupplungszapfen (8b1) sich erstreckt oder, in einer die Kupplungsausnehmung umgebenden Hülsenwand eine Lesevorrichtung (10b) zum Auslesen von in dem Instrument (2) gespeicherten Identifizierungs- und/oder Betriebsparametematen angeordnet ist.

**Claims**

1. Medical or dental instrument (2) having a memory element (10p) for identification and/or operating parameter data,

wherein the memory element (10p) is designed for overwriting of the data by other data, and wherein the instrument (2) comprises a coupling part, by means of which it may be coupled to a corresponding coupling part of a supply line (4) of a supply device (3) for supplying media to the instrument (2), wherein the coupling part is formed by a coupling recess (8a) disposed at the rear of the instrument (2) or by a coupling pin (8b) projecting from the rear of the instrument,

**characterized in that**

the memory element (10p) is disposed in the sleeve wall (2a) surrounding the coupling recess (8a) and is accessible by the corresponding coupling part from the inner lateral surface of the sleeve wall (2a) or from the rear annular end face of the sleeve wall (2a) or is disposed in the coupling pin (8b) and accessible from the lateral surface of the coupling pin (8b) or is disposed in the coupling pin base behind an annular step face and accessible from the annular step face.

2. Instrument according to claim 1,
**characterized in that**
the memory element (10p) comprises contact means (10q) for the input and/or output of data or means of effecting the contactless input and/or output of data.

3. Use of an instrument (2) according to claim 1 or 2, wherein the instrument (2) is to run through at least one operating phase and at least one maintenance phase,

wherein at least one parameter from the operating phase is written into the memory element (10p) and read out for control of the maintenance phase,

and/or at least one parameter from the maintenance phase is written into the memory element (10p) and read out for control of the operating phase.

4. Supply device (3) for supplying media to at least one medical or dental instrument (2) according to one of claims 1 to 2, wherein the supply device (3) is computer-controlled and there extends from the supply device (3) a supply line (4), on the end of which is disposed a coupling part, by means of which the supply line (4) is connectable to the instrument (2), and wherein disposed in the region of the coupling part is a data receiver for receiving identification and/or operating data, which are stored in a memory element (10p) of the instrument (2),

**characterized in that** the coupling part is formed by a coupling pin (8b) that projects from the supply line (4) or by a coupling recess and the data receiver is disposed in the coupling pin (8b) or behind the annular shoulder face, from which the coupling pin extends, or in a sleeve wall surrounding the coupling recess.

5. Supply device according to claim 4,
**characterized in that**
the data receiver is also a data transmitter (100) for transmitting data to the memory element (10p).

6. Maintenance device (31; 32) for at least one medical or dental instrument (2) according to one of claims 1 to 3,
**characterized by**
that it comprises a coupling part, by means of which the instrument (2) may be coupled to the maintenance device (31; 32),
wherein the coupling part is formed by a projecting coupling pin (8b1) or by a coupling recess and a reading device (10b) for reading out identification and/or operating parameter data stored in the instrument (2) is disposed in the coupling pin (8b1) or behind the annular shoulder face, from which the coupling pin (8b1) extends, or in a sleeve wall surrounding the coupling recess.

**Revendications**

1. Instrument médical ou de médecine dentaire (2) comportant un élément d'enregistrement (10p) de données de paramètres d'identification et/ou d'exploitation,
l'élément d'enregistrement (10p) étant configuré pour l'écrasement de données par d'autres données et l'instrument (2) présentant une partie de couplage avec laquelle il peut être couplé à une partie de couplage correspondante d'un câble d'alimentation (4) d'un appareil d'alimentation (3) pour alimenter l'instrument (2) en media,
la partie de couplage étant formée par un évidement de couplage (8a) disposé à l'arrière de l'instrument (2) ou par un tenon de couplage (8b) dépassant de l'arrière de l'instrument
**caractérisé en ce que** l'élément d'enregistrement (10p) est disposé dans la paroi de gaine entourant l'évidement de couplage (8a) et est accessible pour la partie de couplage correspondante de la surface de revêtement interne de la paroi de gaine (2a) ou de la surface annulaire arrière de la paroi de gaine (2a) ou est disposé dans le tenon de couplage (8b) et est accessible du revêtement de surface du tenon de couplage (8b) ou est disposé à la base du tenon de couplage à l'arrière d'un pallier circulaire et est accessible depuis la surface de pallier circulaire.

2. Instrument selon la revendication 1, **caractérisé en ce que** l'élément d'enregistrement (10p) présente des moyens de contact (10q) pour la réception et/ou l'émission de données ou des moyens pour la réception et/ou l'émission sans contacts de données.

3. Utilisation d'un instrument (2) selon la revendication 1 ou 2, au cours de laquelle celui-ci doit passer par au moins une phase d'utilisation et au moins une phase d'entretien,
dans l'élément d'enregistrement (10p) étant inscrit au moins un paramètre de la phase d'utilisation et celui-ci est lu pour commander la phase d'entretien, et/ou dans l'élément d'enregistrement (10p) étant inscrit au moins un paramètre de la phase d'entretien et celui-ci est lu pour commander la phase d'utilisation.

4. Appareil d'alimentation (3) pour alimenter au moins un instrument médical ou un instrument de médecine dentaire (2) selon l'une des revendications 1 à 2 avec des médias, l'appareil d'alimentation (3) étant commandé par ordinateur et un câble d'alimentation (4) s'étendant de l'appareil d'alimentation (3) à l'extrémité duquel une partie de couplage est disposée, avec lequel le câble d'alimentation (4) peut être lié à l'instrument (2) et dans lequel dans la zone de la partie de couplage se trouve un récepteur de données pour recevoir des données d'identifications et/ou d'exploitation qui sont enregistrées dans un élément d'enregistrement (10p) de l'instrument (2),
**caractérisé en ce que**
la partie de couplage est formée par un tenon de couplage (8b) dépassant du câble d'alimentation (4) ou un évidement de couplage et le récepteur de données s'étend dans le tenon de couplage (8b) ou à l'arrière de la surface d'épaulement circulaire de laquelle s'étend le tenon de couplage, ou est disposé dans la paroi de gaine entourant l'évidement de couplage.

5. Appareil d'alimentation selon la revendication 4, **caractérisé en ce que**
le récepteur de données est un émetteur de données (10o) pour transmettre des données sur l'élément d'enregistrement (10p).

6. Appareil médical (31, 32) d'au moins un instrument médical ou de médecine dentaire (2) selon l'une des revendications 1 à 3,
**caractérisé en ce qu'**il présente une partie de couplage par laquelle l'instrument de couplage (2) peut être couplé à l'appareil d'entretien (31 : 32),
la partie de couplage étant formée par un tenon de couplage proéminent (8b1) ou un évidement de couplage et s'étendant dans le tenon de couplage (8b1) ou à l'arrière de la surface d'épaulement de laquelle s'étend le tenon de couplage (8b1) ou un dispositif de lecture (10b) est disposé dans une paroi de gaine entourant l'évidement de couplage pour lire des données de paramètres d'identification et/ou d'exploitation enregistrés dans l'instrument (2).

Fig. 1

Fig. 2

Fig. 3

Fig. 4

Fig. 5

**Fig. 6**

**Fig. 7**

**Fig. 9**

**Fig. 8**

Fig. 12

Fig. 13

Fig. 14a

Fig. 14b

Fig. 10

Fig. 11

Fig. 15

Fig. 16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4410276 A1
- DE 4220522 A1
- DE 19729177 A1
- DE 19913962 A1
- WO 986338 A